# EUROPEAN PATENT APPLICATION

(11) **EP 1 790 331 A1**
(43) Date of publication of application: **30.05.2007**
(21) Application number: 06005748.6
(22) Date of filing: 21.03.2006
(51) Int. Cl.: A61K 9/08, A61K 31/675, A61P 9/00

(54) **Use of pyridoxal-5-phosphate for the treatment of cardiovascular and related pathologies**

(30) Priority: 28.11.2005 US 740970 P; 23.12.2005 US 753853 P
(71) Applicant: Medicure International Inc., Holetown, St.-James (BB)
(72) Inventor: Friesen, Albert D., Winnipeg Manitoba R3P 1P4 (CA)
(74) Representative: Vossius & Partner

(57) **Abstract**

Compositions and methods for treatment and prevention of hypertrophy, congestive heart failure, ischemic heart disease, organ ischemia, tissue ischemia, acute coronary syndrome, unstable angina, ischemia-related conditions, such as ischemia reperfusion injury and cellular dysfunction, are described. Methods are directed to administering pharmaceutical compositions containing at least one compound selected from pyridoxal-5'-phosphate, pyridoxine, pyridoxal, and pyridoxamine, in (1) an oral dosage of 100-1000 mg per day, for example, 200-300 mg/day, 250 mg/day or 750 mg/day or (2) a parenteral dosage of 1.88 to 18.76 mg/day, for example, 3.76 to 5.64 mg/day, 4.69 mg/day, or 14.07 mg/day. Methods are also suitable for preventing these conditions after a traumatic event, such as invasive heart surgery.

## Description

### Cross-Reference to Related Applications

This application claims priority from United States Patent Application Serial No. 60/740,970 filed November 29, 2005 and United States Patent Application Serial No. 60/753,853 filed December 23, 2005.

### Field of the Invention

This invention relates to a method of treating hypertrophy, congestive heart failure, ischemic heart disease, myocardial infarction, acute coronary syndrome, unstable angina, ischemia in various organs and tissues, ischemia reperfusion injuries in various organs and tissues, and to treating cellular dysfunction, including arrhythmia and heart failure subsequent to myocardial infarction.

### Background

Heart failure is the pathophysiological state in which the heart is unable to pump blood at a rate commensurate with the requirement of the metabolizing tissues or can do so only from an elevated filling pressure (increased load). When this condition leads to excess fluid accumulation, such as peripheral edema, it is referred to as congestive heart failure. Heart failure can occur subsequent to myocardial infarction, which is necrosis of a region of the myocardium caused by an interruption in the supply of blood to the heart, usually as a result of occlusion of a coronary artery.

When an excessive pressure or volume load is imposed on the ventricle, myocardial hypertrophy develops, providing a compensatory mechanism that permits the ventricle to sustain an increased load. However, a ventricle subjected to an abnormally elevated load for a prolonged period may fail to maintain compensation despite the presence of ventricular hypertrophy and pump failure may ultimately occur. When heart failure occurs in a ventricle, it is sometimes referred to as a ventricular failure.

Ischemia is caused when an organ or a part of the body fails to receive a sufficient blood supply. An organ that is deprived of a blood supply is said to be hypoxic. An organ will become hypoxic even when the blood supply temporarily ceases, such as during a surgical procedure or during temporary artery blockage. Ischemia leads to structural and functional abnormalities, such as arrhythmias, cell death, and ventricular remodeling.

When the organ affected is the heart, this condition is known as ischemic heart disease, or myocardial ischemia.

Myocardial ischemia initially leads to abnormal electrical activity, which may generate an arrhythmia. When myocardial ischemia is of sufficient severity and duration, cell injury may progress to cell death i.e., myocardial infarction-and subsequently to heart failure, hypertrophy, or congestive heart failure. Other organs and tissues can be affected, such as the kidney, colon, panchreas, an arm, leg, etc. Ischemia of various organs and tissues is well known and characterized, and can cause structural and functional abnormalities including failure of the organ.

When blood flow resumes to an organ after temporary cessation, this is known as ischemic reperfusion of the organ. For example, reperfusion of an ischemic myocardium may counter the effects of coronary occlusion, a condition that leads to myocardial ischemia. Ischemic reperfusion to the myocardium may lead to reperfusion arrhythmia or reperfusion injury. The severity of reperfusion injury is affected by numerous factors, such as, for example, duration of ischemia, severity of ischemia, and speed of reperfusion. Conditions observed with ischemia reperfusion injury include neutrophil infiltration, necrosis, and apoptosis.

Lack of blood flow to the brain, through ischemia or any other heart-related disease, can cause stroke, which may lead to sensorimotor abnormalities such as paralysis, which can be permanent.

Ischemia and other heart diseases are often associated with angina, a heart condition marked by paroxysms of chest pain due to reduced oxygen to the heart.

Acute Coronary Syndrome is a term used to describe cardiovascular irregularities; the term encompasses a range of thrombolitic coronary artery diseases, including unstable angina, Q-wave myocardial infarction and non-Q-wave myocardial infarction.

Coronary artery bypass grafting (CABG) effectively relieves angina, results in longer survival, and a better quality of life in specific subgroups of patients with obstructive coronary artery disease. Due to the high incidence of coronary artery disease worldwide, as well as the effectiveness of this surgical procedure, CABG surgery makes up one of the top ten most frequently performed procedures in North America and Europe. In the year 2000, the total volume of bypass surgery was over 280,000 in the 15 European Union countries according to the European Heart Survey and the National Registries of Cardiovascular Diseases and Patient Management. In the United States, it is estimated that over 700,000 CABG procedures are performed per year.

Despite the benefits of CABG surgery, patients undergoing these procedures may also suffer serious adverse outcomes including operative mortality, myocardial infarction, acute coronary syndrome, unstable angina, ventricular failure, life-threatening arrhythmia, renal insufficiency, and stroke. Some of the proposed causes of cardiovascular morbidity and mortality after CABG include perioperative ischemia, inadequate myocardial protection, and reperfusion injury. The impact of these serious complications is significant. Incidence rates of death and MI following CABG surgery range from 5% to 12% depending on risk status. Results from large clinical trials have recently demonstrated the importance of neurologic deficits as a problematic outcome of CABG. These deficits include impairment of memory, psychomotor, visuospatial, attention, and language abilities as measured by neuropsychological testing as well as the sensorimotor abnormalities associated with stroke.

It is difficult to assess whether myocardial infarction has, in fact, occurred. The "gold standard" for measuring myocaridal infarction is a biochemical assay which looks at creatine kinase-MB (CK-MB) levels in the blood. Elevated CK-MB levels are correlated with myocardial infarction. For example, a CK-MB level of 10X ULN (50 ng/ml) or above is often used as an indicator of myocardial ischemia. Other experts, and other studies, look at varying "cut-offs" of CK-MB elevation as indicative of the existence of myocardial ischemia, with 50, 70, and 100 ng/ml cut-offs appearing most frequently in the literature.

Pyridoxal-5'-phosphate (3-hydroxy-2-methyl-5-[(phosphonooxy)methyl]-4-pyridine-carboxaldehyde, or "P5P") is a naturally occurring metabolite of pyridoxine (Vitamin B6) and is formed in mammalian cells by phosphorylation and oxidation reactions. A recent evaluation demonstrated that P5P inhibits adenosine triphosphate (ATP) induced calcium ion influx into cells. Results suggest that this action is due to an inhibition of purinergic receptors known as P2 purinoceptors. It is hypothesized that P5P might prevent or reduce tissue damage during ischemia and reperfusion episodes by blocking calcium influx.

P5P can be chemically synthesized in a number of ways, for example, by the action of ATP on pyridoxal, by the action of phosphorus oxychloride on pyridoxal in aqueous solution, and by phosphorylation of pyridoxamine with concentrated phosphoric acid followed by oxidation.

P5P and other related compounds such as pyridoxine, pyridoxal and pyridoxamine, have been previously shown to be effective in the treatment of hypertrophy, congestive heart failure, ischemic heart disease, ischemia reperfusion injuries in an organ, and arrhythmia and contractile dysfunction subsequent to myocardial infarction. P5P has also been used therapeutically as an enzyme cofactor vitamin.

P5P is described in US Patent 6,043,259 herein incorporated by reference, as having an effective enteral or parenteral dosage range of about 0.5 to about 100 mg/kg of patient body weight, for treating hypertrophy, hypertension, congestive heart failure, ischemic heart disease, ischemia reperfusion injury and cellular dysfunction.

### Summary of the Invention

The present invention includes methods and compositions for treating cardiovascular diseases and diseases related thereto. In one aspect, the invention includes a method for treating hypertrophy, myocardial ischemia, organ ischemia, such as kidney ischemia, tissue ischemia, acute coronary syndrome, unstable angina, myocardial infarction, congestive heart failure, ischemic heart disease, ischemia reperfusion injury and cellular dysfunction (including arrhythmia, heart failure subsequent to myocardial infarction, cell death, and ventricular remodeling) in mammals that includes orally administering to the mammal a therapeutic amount in a range of about 1-10 mg/kg of the mammal's body weight per day of a compound selected from the group consisting of pyridoxine, pyridoxal-5'-phosphate, pyridoxal, and pyridoxamine. In another aspect, administration can treat or prevent contractile dysfunction subsequent to myocardial infarction. In another aspect, administration can treat or prevent cerebral infarction (stroke). The cerebral infarction (stroke) can be a cerebral infarction (stroke) occurring subsequent to myocardial infarction.

In another aspect, the invention includes a method of preventing death subsequent to myocardial infarction in a mammal comprising: orally administering to said mammal a therapeutic amount in a range of about 1-10 mg/kg of the mammal's body weight per day of a compound selected from the group consisting of pyridoxine, pyridoxal-5'-phosphate, pyridoxal and pyridoxamine.

In another aspect, the compound can be administered prior to, or following, a heart procedure such as bypass surgery, thrombolysis, CABG and angioplasty.

Another aspect of the invention is the methods as described above wherein the mammal is a human and said oral therapeutic amount is in a range of about 100 mg per day to about 1000 mg per day, about 200 mg per day to about 300 mg per day, about 250 mg per day, or about 750 mg per day.

In another aspect, the invention includes a method for treating hypertrophy, myocardial ischemia, myocardial infarction, congestive heart failure, ischemic heart disease, organ ischemia such as kidney ischemia, tissue ischemia, acute coronary syndrome, unstable angina, ischemia reperfusion injury and cellular dysfunction (including arrhythmia, heart failure subsequent to myocardial infarction, cell death, and ventricular remodeling) in mammals that includes parenterally administering to the mammal a therapeutic amount in a range of about 0.067 to about 0.67 mg/kg of the mammal's body weight per day of a compound selected from the group consisting of pyridoxine, pyridoxal-5'-phosphate, pyridoxal, and pyridoxamine. In another aspect, administration can treat or prevent contractile dysfunction subsequent to myocardial infarction. In another aspect, administration can treat or prevent cerebral infarction (stroke). The cerebral infarction (stroke) can be a cerebral infarction (stroke) occurring subsequent to myocardial infarction.

In another aspect, the invention includes a method of preventing death subsequent to myocardial infarction in a mammal comprising: parenterally administering to said mammal a therapeutic amount in a range of about 0.067 to about 0.67 mg/kg of the mammal's body weight per day of a compound selected from the group consisting of pyridoxine, pyridoxal-5'-phosphate, pyridoxal, and pyridoxamine.

In another aspect, the compound can be administered prior to, or following, a heart procedure such as bypass surgery, thrombolysis, CABG and angioplasty.

Another aspect of the invention is the methods as described above wherein the mammal is a human and said parenteral therapeutic amount is in a range of about 1.88 mg per day to about 18.76 mg per day, about 3.76 mg per day to about 5.64 mg per day, about 4.69 mg per day, or about 14.07 mg per day.

The parenteral administration may be an intravenous injection. The intravenous injection may be either a bolus or a continuous injection. The oral administration may be an enteral administration, such as a liquid, pill or capsule to be swallowed.

Another aspect of the present invention is a composition formulated for oral administration comprising about 100 to about 1000 mg of pyridoxal-5'-phosphate, about 250 mg of pyridoxal-5'-phosphate, about 750 mg of pyridoxal-5'-phosphate, or about 100 to about 1000 mg, about 250 mg, or about 750 mg of any of the other compounds discussed above, optionally further comprising a pharmaceutically acceptable excipient.

Another aspect of the present invention is a composition formulated for parenteral administration comprising about 1.88 mg to 18.76 mg of pyridoxal-5'-phosphate, about 4.69 mg of pyridoxal-5'-phosphate, about 14.07 mg of pyridoxal-5'-phosphate, or about 1.88 mg to 18.76 mg, about 4.69 mg, or about 14.07 mg of any of the other compounds discussed above, optionally further comprising a pharmaceutically acceptable excipient.

Another aspect of the present invention is the use of 100 to 1000 mg of pyridoxine, pyridoxal-5'-phosphate, pyridoxal or pyridoxamine in the preparation of a medicament for the treatment or prevention of hypertrophy, myocardial ischemia, organ ischemia such as kidney ischemia, tissue ischemia, acute coronary syndrome, unstable angina, conjestive heart failure, contractile dysfunction, ischemia reperfusion injury, arrhythmia, or any other disease or condition discussed above.

Another aspect of the present invention is the use of 100 to 1000 mg of pyridoxine, pyridoxal-5'-phosphate, or pyridoxamine in the preparation of a medicament for the treatment or prevention of post-operative complications. The post-operative complication may arise from an operation such as bypass surgery, thrombolysis, CABG, and angioplasty. The post-operative complication may be hypertrophy, myocardial ischemia, organ ischemia such as kidney ischemia, tissue ischemia, acute coronary syndrome, unstable angina, ischemia reperfusion injury, arrhythmia, conjestive heart failure, contractile dysfunction, cerebral infarction (stroke), or death. Between about 200 mg and about 300 mg of the compound may be used. About 250 mg of the compound may be used. About 750 mg of the compound may be used.

Another aspect of the present invention is the use of 1.88 mg to 18.76 mg of pyridoxine, pyridoxal-5'-phosphate, pyridoxal or pyridoxamine in the preparation of a medicament for the treatment or prevention of hypertrophy, myocardial ischemia, organ ischemia such as kidney ischemia, tissue ischemia, acute coronary syndrome, unstable angina, conjestive heart failure, contractile dysfunction, ischemia reperfusion injury, arrhythmia, or any other disease or condition discussed above.

Another aspect of the present invention is the use of 1.88 mg to 18.76 mg of pyridoxine, pyridoxal-5'-phosphate, or pyridoxamine in the preparation of a medicament for the treatment or prevention of post-operative complications. The post-operative complication may arise from an operation such as bypass surgery, thrombolysis, CABG, and angioplasty. The post-operative complication may be hypertrophy, myocardial ischemia, organ ischemia such as kidney ischemia, tissue ischemia, acute coronary syndrome, unstable angina, ischemia reperfusion injury, arrhythmia, conjestive heart failure, contractile dysfunction, cerebral infarction (stroke), or death. Between about 3.76 mg and about 5.64 mg of the compound may be used. About 4.69 mg of the compound may be used. About 14.07 mg of the compound may be used.

In another aspect, the present invention includes methods and compositions for preventing post-operative complications, morbidity and mortality after surgical procedures (such as open heart surgery, for example, CABG), such post-operative complications including hypertrophy, congestive heart failure, ischemic heart disease, organ ischemia such as kidney ischemia, tissue ischemia, acute coronary syndrome, unstable angina, ischemia reperfusion injury and cellular dysfunction (including arrhythmia, heart failure subsequent to myocardial infarction, cell death, and ventricular remodeling) in mammals that includes administering to the mammal a therapeutically effective amount of a compound selected from the group consisting of pyridoxal-5'-phosphate, pyridoxine, pyridoxal, and pyridoxamine.

In another aspect, the invention is directed to a pharmaceutical composition that includes a pharmaceutically acceptable carrier and a therapeutically effective amount, from about 200 to about 1000 mg (oral) or about 1.88 mg to about 18.76 mg (parenteral), of a compound selected from the group consisting of pyridoxal-5'-phosphate, pyridoxine, pyridoxal, and pyridoxamine for treating hypertrophy, congestive heart failure, ischemic heart disease, organ ischemia such as kidney ischemia, tissue ischemia, acute coronary syndrome, unstable angina, ischemia reperfusion injury and cellular dysfunction or for preventing post-operative complications after surgical procedures (such as open heart surgery, for example, CABG), such post-operative complications including hypertrophy, congestive heart failure, ischemic heart disease, organ ischemia such as kidney ischemia, tissue ischemia, acute coronary syndrome, unstable angina, ischemia reperfusion injury and cellular dysfunction (including arrhythmia, heart failure subsequent to myocardial infarction, cell death, and ventricular remodeling).

In another aspect, the invention is directed to a pharmaceutical composition that includes a pharmaceutically acceptable carrier and a therapeutically effective amount, from about 200 to about 300 mg (oral) or about 3.76 mg to about 5.64 mg (parenteral), of a compound selected from the group consisting of pyridoxal-5'-phosphate, pyridoxine, pyridoxal, and pyridoxamine for treating hypertrophy, congestive heart failure, ischemic heart disease, organ ischemia such as kidney ischemia, tissue ischemia, acute coronary syndrome, unstable angina, ischemia reperfusion injury and cellular dysfunction or for preventing post-operative complications after surgical procedures (such as open heart surgery, for example, CABG), such post-operative complications including hypertrophy, congestive heart failure, ischemic heart disease, organ ischemia such as kidney ischemia, tissue ischemia, acute coronary syndrome, unstable angina, ischemia reperfusion injury and cellular dysfunction (including arrhythmia, heart failure subsequent to myocardial infarction, cell death, and ventricular remodeling).

In another aspect, the invention is directed to a pharmaceutical composition that includes a pharmaceutically acceptable carrier and a therapeutically effective amount, of about 250 mg (oral) or about 4.69 mg (parenteral), of a compound selected from the group consisting of pyridoxal-5'-phosphate, pyridoxine, pyridoxal, and pyridoxamine for treating hypertrophy, congestive heart failure, ischemic heart disease, organ ischemia such as kidney ischemia, tissue ischemia, acute coronary syndrome, unstable angina, ischemia reperfusion injury and cellular dysfunction, or for preventing post-operative complications after surgical procedures (such as open heart surgery, for example, CABG), such post-operative complications including hypertrophy, congestive heart failure, ischemic heart disease, organ ischemia such as kidney ischemia, tissue ischemia, acute coronary syndrome, unstable angina, ischemia reperfusion injury and cellular dysfunction (including arrhythmia, heart failure subsequent to myocardial infarction, cell death, and ventricular remodeling).

### Detailed Description

The present inventors have surprisingly and paradoxically found that, P5P is effective throughout the dose range disclosed in the prior art. However, P5P is *more* effective at the lower end of this dosage range, as compared to the higher end of the dosage range, especially when certain, clinically accepted, indicators of myocardial ischemia are used. The inventors have surprisingly found that an oral administration of a dosage range of 200-300 mg per day, for example, 250 mg per day, in humans, is more effective at prevention of post-CABG cardiovascular morbidity and mortality than higher dosages such as 750 mg per day. This finding defies the expectations of the current state of the art and the biochemical methods of action of P5P currently hypothesized. The inventors have also found that oral dosages of 250 mg per day and 750 mg per day are effective at prevention of post-CABG cardiovascular morbidity and mortality in humans, and therefore hypothesize that a dosage range of 200 mg per day to 1000 mg per day, administered orally, or a pharmacokinetically equivalent dosage administered parenterally, for example, an IV dosage providing a mean AUC₍₀₋₂₄ₕ₎ of about 2 to about 11 ug.h/ml should be effective in reducing post-operative complications, morbidity and mortality, and should be appropriate post- operative treatment protocol for any form of invasive heart surgery, such as CABG. The IV dosage may also be , 2.08-3.12 ± 1.04 ug.h/ml, 2.60 ± 0.87 ug.h/ml, or 7.80 ± 2.61 ug.h/ml.

The present inventors have found that, for an average 70kg person, a total IV dose of 4.69 mg is roughly equivalent to a 250 mg pill-form enteral administration, a total IV dose of 14.07 mg is roughly equivalent to a 750 mg pill-form enteral administration, and an IV dose range of 1.88-18.76 mg is roughly equivalent to a pill-form enteral administration range of 100-1000 mg.

Relatively low concentrations of P5P can be used advantageously in the treatment of the above-identified diseases and conditions. "Treatment" and "treating" as used herein include preventing, inhibiting, and alleviating cardiovascular diseases and related symptoms as well as healing the ischemia-related conditions or symptoms thereof affecting mammalian organs and tissues. Thus a composition of the present invention can be administered in a therapeutically effective amount to a patient before, during, and after any above-mentioned condition arises. As an example, for instance, a composition of the present invention can be administered prior to ischemia to prevent, inhibit, or protect against ischemia reperfusion injuries and cellular dysfunction of organs and tissues, such as kidney ischemia. Alternatively, a composition of the invention can be administered during or following ischemia (including during or following reperfusion) to alleviate or heal ischemia reperfusion injuries and cellular dysfunction of organs and tissues. In one embodiment, a composition of the invention can be administered after invasive surgery which is known to trigger these injuries, such as a CABG treatment.

In one aspect, the invention is directed to a method of treating, preventing, or protecting against, hypertrophy, congestive heart failure, ischemic heart disease, ischemia reperfusion injury, organ ischemia such as kidney ischemia, tissue ischemia, acute coronary syndrome, unstable angina, ischemia-related injuries, and cellular dysfunction in mammals comprising administering to the mammal a therapeutic amount of a compound selected from the group consisting of pyridoxal-5'-phosphate, pyridoxine, pyridoxal and pyridoxamine. Cellular dysfunction may include an arrhythmia of the heart or heart failure resulting from myocardial infarction. A "therapeutic amount" as used herein includes a prophylactic amount, for example, an amount effective for preventing or protecting against ischemia-related conditions, and amounts effective for alleviating or healing ischemia-related conditions.

Orally administering a therapeutic amount of the compound for treating hypertrophy, congestive heart failure, ischemic heart disease, organ ischemia such as kidney ischemia, tissue ischemia, acute coronary syndrome, unstable angina, ischemia reperfusion injury and cellular dysfunction preferably is in the range of about 1-10 mg/kg of a patient's body weight, such as in the range of about 100-1000 mg, per daily dose, the range of about 200-300 mg per daily dose, or about 250 mg per daily dose, or, optionally, about 750 mg per daily dose.

Parenterally administering a therapeutic amount of the compound for treating hypertrophy, congestive heart failure, ischemic heart disease, organ ischemia such as kidney ischemia, tissue ischemia, acute coronary syndrome, unstable angina, ischemia reperfusion injury and cellular dysfunction preferably is in the range of about 0.067 to 0.67 mg/kg of a patient's body weight, such as in the range of about 1.88 to about 18.76 mg per daily dose, the range of about 3.76 - 5.64 mg per daily dose, or about 4.69 mg per daily dose, or, optionally, about 14.07 mg per daily dose.

The compound may be administered for periods of short and long durations depending on the condition treated.

A therapeutic amount of the compound for particularly treating ischemia-related conditions can be administered before, during, or following ischemia (including during or following reperfusion), as well as continually for some period spanning from pre- to post-ischemia. For example, a compound may be administered prior to heart procedures, including bypass surgery, thrombolysis, and angioplasty, and prior to any other procedures that require interruption and then resumption of blood flow to any organ. The compound can also be taken during or after said heart procedure. Additionally, a compound may be taken on a regular basis to protect against cellular dysfunction arising from arrhythmia and heart failure.

By way of illustration, the following describes administration to a human of a pharmaceutical composition containing P5P for ischemia. When a human is presented for a heart procedure, for example, bypass surgery, CABG, thrombolysis, or angioplasty, or for a procedure requiring interruption of blood flow to any organ, an aqueous solution comprising P5P in a therapeutic amount can be given intravenously, immediately prior to surgery and then throughout a patient's hospitalization. Alternatively, a pharmaceutical composition comprising P5P can be given immediately prior to surgery and then continuously for up to 30 days following surgery.

Similarly, a human may be administered an oral or parenteral dose of P5P beginning with the onset of symptoms of ischemia-related conditions through the surgical procedure. Furthermore, a human at risk for arrhythmia or heart failure may be administered a regular oral or patenteral dose of P5P to protect against cellular dysfunction.

A pharmaceutical composition of the present invention is directed to a composition suitable for the treatment of hypertrophy, congestive heart failure, ischemic heart disease, organ ischemia such as kidney ischemia, tissue ischemia, acute coronary syndrome, unstable angina, ischemia reperfusion injury and cellular dysfunction. A pharmaceutical composition comprises a pharmaceutically acceptable carrier and a compound selected from the group consisting of pyridoxal-5'-phosphate, pyridoxine, pyridoxal and pyridoxamine. A pharmaceutically acceptable carrier includes, but is not limited to, physiological saline, ringers, phosphate buffered saline, and other carriers known in the art. Pharmaceutical compositions may also include stabilizers, antioxidants, colorants, and diluents. Pharmaceutically acceptable carriers and additives are chosen such that side effects from the pharmaceutical compound are minimized and the performance of the compound is not canceled or inhibited to such an extent that treatment is ineffective. A selected compound may be P5P. Advantageous pharmaceutical compositions include those that are made specifically for the oral treatment dosage levels discovered herein, for example, a pharmaceutical composition for oral administration comprising about 250 mg of P5P or an other compound of the invention, a pharmaceutical composition for oral administration comprising about 750 mg of P5P or another compound of the invention, or a pharmaceutical composition for oral administration comprising about 100 to about 1000 mg of P5P or another compound of the invention.

Other advantageous pharmaceutical compositions include those that are made specifically for the parenteral treatment dosage levels discovered herein, for example, a pharmaceutical composition for parenteral administration comprising about 4.69 mg of P5P or an other compound of the invention, a pharmaceutical composition for parenteral administration comprising about 14.07 mg of P5P or another compound of the invention, or a pharmaceutical composition for parenteral administration comprising about 1.88 to about 18.76 mg of P5P or another compound of the invention.

Pharmaceutical compositions may be administered orally and parenterally. Parenteral administration includes subcutaneous, intramuscular, intradermal, intramammary, intravenous, and other administrative methods known in the art. Parenteral administration may be bolus or continuous infusion. Oral administration includes enteral administration of solution, tablets, sustained release capsules, enteric coated capsules, and syrups. When administered, the pharmaceutical composition should be at or near body temperature.

Methods of preparing pharmaceutical compositions containing a pharmaceutically acceptable carrier and a therapeutic compound selected from P5P, pyridoxine, pyridoxal and pyridoxamine are known to those of skill in the art. As an illustration, a method of preparing a pharmaceutical composition containing P5P will be described.

The invention also includes pharmaceutically acceptable salts of the compounds of the invention. The compounds of the invention are capable of forming both pharmaceutically acceptable acid addition and/or base salts. Pharmaceutically acceptable acid addition salts of the compounds of the invention include salts derived from nontoxic inorganic acids such as hydrochloric, nitric, phosphoric, sulfuric, hydrobromic, hydriodic, hydrofluoric, phosphorous, and the like, as well as the salts derived from nontoxic organic acids, such as aliphatic mono- and di-carboxylic acids, phenyl-substituted alkanoic acids, hydroxy alkanoic acids, alkanedioic acids, aromatic acids, aliphatic and aromatic sulfonic acids, etc. Such salts thus include sulfate, pyrosulfate, bisulfate, sulfite, bisulfite, nitrate, phosphate, monohydrogenphosphate, dihydrogenphosphate, metaphosphate, pyrophosphate, chloride, bromide, iodide, acetate, trifluoroacetate, propionate, caprylate, isobutyrate, oxalate, malonate, succinate, suberate, sebacate, fumarate, maleate, mandelate, benzoate, chlorobenzoate, methylbenzoate, dinitrobenzoate, phthalate, benzenesulfonate, toluenesulfonate, phenylacetate, citrate, lactate, maleate, tartrate, methanesulfonate, and the like. Also contemplated are salts of amino acids such as arginate and the like and gluconate, galacturonate, n-methyl glucamine, etc. (see Berge et al., J. Pharmaceutical Science, 66: 1-19 (1977). The term "pharmaceutically acceptable salts" also includes any pharmaceutically acceptable base salt including, but not limited to, amine salts, trialkyl amine salts and the like. Such salts can be formed quite readily by those skilled in the art using standard techniques.

The acid addition salts of the basic compounds are prepared by contacting the free base form with a sufficient amount of the desired acid to produce the salt in the conventional manner. The free base form may be regenerated by contacting the salt form with a base and isolating the free base in the conventional manner. The free base forms differ from their respective salt forms somewhat in certain physical properties such as solubility in polar solvents, but otherwise the salts are equivalent to their respective free base for purposes of the present invention. Base salts are formed with metals or amines, such as alkali and alkaline earth metals or organic amines. Examples of metals used as cations include, but are not limited to, sodium, potassium, magnesium, and calcium. Examples of suitable amines are N,N'-dibenzylethylenediamine, chloroprocaine, choline, diethanolamine, ethylenediamine, N-methylglucamine, and procaine.

Some of the compounds described herein contain one or more asymmetric centers and may thus give rise to enantiomers, diastereomers, and other stereoisomeric forms which may be defined in terms of absolute stereochemistry as (R)- or (S)-. The present invention is meant to include all such possible diastereomers and enantiomers as well as their racemic and optically pure forms. Optically active (R)- and (S)- isomers may be prepared using chiral synthons or chiral reagents, or resolved using conventional techniques. When the compounds described herein contain centers of geometric asymmetry, and unless specified otherwise, it is intended that the compounds include both E and Z geometric isomers. Likewise all tautomeric forms are intended to be included.

U.S. Patent No. 6,339,085 and U.S. Patent No. 6,861,439 are herein incorporated by reference.

Generally, a P5P solution may be prepared by simply mixing P5P with a pharmaceutically acceptable solution, for example, buffered aqueous saline solution at an acidic or alkaline pH (because P5P is essentially insoluble in water, alcohol, and ether), at a temperature of at least room temperature and under sterile conditions. Preferably, a P5P solution is prepared immediately prior to administration to the mammal. However, if a P5P solution is prepared at a time more than immediately prior to the administration to the mammal, the prepared solution should be stored under sterile, refrigerated conditions. Furthermore, because P5P is light sensitive, a P5P solution should be stored in containers suitable for protecting a P5P solution from the light, such as amber-colored vials or bottles. Each amber colored vial or bottle can contain 250 mg of P5P or another compound of the invention, or 750 mg of P5P or another compound of the invention, or from about 200 to about 1000 mg of P5P or another compound of the invention, for oral use. Alternatively, for parenternal use, each amber colored bottle or vial can contain about 4.69 mg of P5P or another compound of the invention, about 14.07 mg of P5P or another compound of the invention, or from about 1.88 to about 18.76 mg of P5P or another compound of the invention.

According to the embodiment of invention, P5P, pyridoxine, pyridoxal, and pyridoxamine appropriately administered can have previously unexpected, highly beneficial effects in treating hypertrophy, congestive heart failure, ischemia, heart disease, organ ischemia such as kidney ischemia, tissue ischemia, acute coronary syndrome, unstable angina, or ischemia reperfusion injuries in mammals and in treatment of heart dysfunction subsequent to coronary occlusion, at lower concentrations of treatment than what was previously taught. According to embodiments of the invention, lower concentrations of P5P may have paradoxically higher effectiveness, when compared to higher concentrations of P5P previously taught. For example, P5P administered orally at a concentration of 250 mg/day was found to have higher effectiveness at reducing post-CABG morbidity and mortality than concentrations of 750 mg/day when a CK-MB level of greater than the pre-defined level of 50 ng/ml or a post-hoc analysis of 70 ng/ml is used as an indicator of myocardial ischemia. Concentrations of 250 mg/day (orally administered) and of 750 mg/day (orally administered) were both found to be effective at reducing post-CABG morbidity and mortality when a CK-MB level or greater than a post-hoc analysis of 100 ng/ml was used as an indicator of myocardial ischemia.

For illustrative purposes, a beneficial effect of administering P5P is demonstrated in the specific examples detailed below. Although the examples below discuss "high risk" patients, the results can be extended to all patients, regardless of risk, and, as such, the invention is useful for all patients, regardless of risk.

### Examples

### Experiment 1: Oral Treatment in Post-CABG patients

A randomized, double-blind placebo-controlled, dose-ranging, parallel-arm multi-center study was undertaken, on high-risk patients undergoing CABG surgery with cardiopulmonary bypass.

Patients were identified as "high risk" if they had two or more of the following risk factors:
- Age greater than 65 years;
- Current smoker;
- History of diabetes mellitus requiring treatment other than diet;
- Evidence of left ventricular dysfunction or congestive heart failure;
- History of a previous non-disabling stroke, transient ischemic attack, or carotid endarterectomy;
- Urgent CABG intervention defined as the need to stay in the hospital (although the patient may be operated on whithin a normal scheduling routine);
- History of myocardial infarction that occurred more than 48 hours but less than 6 weeks prior ato CABG surgery;
- Prior peripheral artery surgery or angioplasty;
- Moderate renal dysfunction defined as creatinine > 133 micromol/L (1.5 mg/dl), but < 250 micromol/L (2.8 mg/dl); and
- Presence of at least one asymptomatic carotid artery stenosis (>50%) either in one or two carotid arteries.

Approximately 900 high risk pre-CABG patients in 42 different treatment centers in North America were screened and randomized to three groups of approximately 300 patients each, prior to their bypass surgery, as follows. Patients were either placed in a control group (placebo), treated orally with 250 mg/day of P5P, (250 mg/day), or treated orally with 750 mg/day of P5P (750 mg/day).

The first dose of study medication was administered at 3-10 hours prior to CABG surgery. In the event of surgery delay or rescheduling, a second pre-operative dose of P5P was administered so that all patients received study medication 3-10 hours before surgery. Treatment continued for 30 days after surgery (post operative day (POD) 30). Patients received follow-up evaluations up to and including postoperative day (POD) 4, on POD 30 and on POD 90.

Patients were measured for combined incidence of cardiovascular death, nonfatal myocardial infarction (MI), and nonfatal cerebral infarction up to and including post-operative day 30 (POD 30). Patients were also measured for nonfatal myocardial infarction alone.

All deaths without an identifiable non-cardiovascular cause were considered of cardiovascular origin.

Cerebral infarction (stroke) was defined clinically as any new sudden onset focal neurological deficit lasting at least 24 hours as assessed by a neurologist and after neuroimaging (computed tomography [CT] brain scan or magnetic resonance imaging [MRI]) had excluded an intracerebral hemorrhage. All patients suspected of having a stroke or transient ischemic attack (TIA) received a neurological examination (conducted by a neurologist or internist with expertise in cerebral vascular disease) within 24 hours of onset of symptoms. All patients suspected of having a stroke were submitted to cerebral imaging.

When determining whether a patient had myocardial infarction, the following definitions were used:
- A peak creatine kinase - myocardial band (CK-MB) above a certain threshold.
   Since different experts and different prior art clinical trials used different cut-offs for this threshold, three different thresholds were used to determine whether a patient had myocardial infarction - a post-hoc cut off threshold of 100 ng/ml (a peak CK-MB of 100 ng/ml or greater on days up to and including POD 4), a post-hoc cut off threshold of 70 ng/ml, and a predefined cut off threshold of 50 ng/ml was used;
- A new q-wave evidence of myocardial infarction along with CK-MB of 35 ng/ml or above on days up to and including POD 4;
- A peak CK-MB of 5x ULN (25 ng/ml) or above occurring after POD 4;
- A new q-wave evidence of myocardial infarction that was not present at POD 4; or
- A q-wave or non-q-wave myocardial infarction as identified by the investigator and confirmed by the Clinical Endpoint Committee.

### A. Myocardial Infarction Rates

### CK-MB cut-off of 100 ng/ml

Oral treatment of both 250 mg/day and 750 mg/day were highly effective in reducing instances of myocardial infarction. See Table 1.

**Table 1: Frequencies of myocardial infarction at POD 30**

| | Placebo | 250 mg/day P5P | 750 mg/day P5P |
|---|---|---|---|
| Myocardial Infarction | 43 (14.4%) | 23 (7.6%) | 23 (7.6%) |
| No myocardial infarction | 256 (85.6%) | 278 (92.4%) | 278 (92.4%) |

### Using CK-MB cut-off of 70 ng/ml

Oral treatment of 250 mg/day was highly effective in reducing instances of myocardial infarction. Oral treatment of 750 mg/day was effective in reducing instances of myocardial infarction, but was not as effective as treatment of 250 mg/day. See Table 2.

**Table 2: Frequencies of myocardial infarction at POD 30**

| | Placebo | 250 mg/day P5P | 750 mg/day P5P |
|---|---|---|---|
| Myocardial Infarction | 54 (18.1%) | 34 (11.3%) | 38 (12.6%) |
| No myocardial infarction | 245 (81.9%) | 267 (88.7%) | 263 (87.4%) |

### Using CK-MB cut-off of 50 ng/ml

Oral treatment of 250 mg/day was highly effective in reducing instances of myocardial infarction. Oral treatment of 750 mg/day was not effective in reducing instances of myocardial infarction. See Table 3.

**Table 3: Frequencies of myocardial infarction at POD 30**

| | Placebo | 250 mg/day P5P | 750 mg/day P5P |
|---|---|---|---|
| Myocardial Infarction | 69 (23.1%) | 59 (19.6%) | 71 (23.6%) |
| No myocardial infarction | 230 (76.9%) | 242 (80.4%) | 230 (76.4%) |

### B. Combined Incident / Composite Endpoint Rates

Frequencies of combined incidences (including myocardial infarction, cerebral infarction, or death), by treatment group, were as follows:

### CK-MB cut-off of 100 ng/ml

Oral treatment of either 250 mg/day or 750 mg/day was highly effective in reducing total incidents. Oral treatment of 250 mg/day was marginally better at reducing total incidents as compared to treatment of 750 mg/day. See Table 4.

**Table 4: Frequencies of myocardial infarction, cerebral infarction, or death, at POD 30**

| | Placebo | 250 mg/day P5P | 750 mg/day P5P |
|---|---|---|---|
| Myocardial infarction, cerebral infarction or death | 49 (16.4%) | 31 (10.3%) | 34 (11.3%) |
| No myocardial infarction, cerebral infarction or death | 250 (83.6%) | 270 (89.7%) | 267 (88.7%) |

### CK-MB cut-off of 70 ng/ml

Oral treatment of either 250 mg/day or 750 mg/day was highly effective in reducing total incidents. Oral treatment of 250 mg/day was better at reducing total incidents than treatment of 750 mg/day. See Table 5.

**Table 5: Frequencies of myocardial infarction, cerebral infarction, or death, at POD 30**

| | Placebo | 250 mg/day P5P | 750 mg/day P5P |
|---|---|---|---|
| Myocardial infarction, cerebral infarction or death | 60 (20.1%) | 41 (13.6%) | 47 (15.6%) |
| No myocardial infarction, cerebral infarction or death | 239 (79.9%) | 260 (86.4%) | 254 (84.4%) |

### CK-MB cut-off of 50 ng/ml

Oral treatment of 250 mg/day was highly effective in reducing total incidents.
Oral treatment of 750 mg/day did not reduce total incidents. See Table 6.

**Table 6: Frequencies of myocardial infarction, cerebral infarction, or death, at POD 30**

| | Placebo | 250 mg/day P5P | 750 mg/day P5P |
|---|---|---|---|
| Myocardial infarction, cerebral infarction or death | 75 (25.1%) | 65 (21.6%) | 77 (25.6%) |
| No myocardial infarction, cerebral infarction or death | 224 (74.9%) | 236 (78.4%) | 224 (74.4%) |

### Experiment 2: Parenteral Treatment in Post-CABG patients

The randomized, double-blind placebo-controlled, dose-ranging, parallel-arm multi-center study on high-risk patients undergoing CABG surgery with cardiopulmonary bypass of Experiment 1 is repeated using a protocol as described in Experiment 1, with the difference that the placebo/drug is administered intravenously, in an intravenous dose pharmacokinetically approximating the dosage given enterally in Experiment 1.

Approximately 900 high-risk pre-CABG patients are screened and randomized to 3 groups of approximately 300 patients each, prior to their bypass surgery, as follows. Patients are either placed in a control group (placebo), treated intravenously with 4.69 mg/day of P5P, or treated intravenously with 14.07 mg/day of P5P.

Patients are identified as "high risk" if they had two or more of the following risk factors:
- Age greater than 65 years;
- Current smoker;
- History of diabetes mellitus requiring treatment other than diet;
- Evidence of left ventricular dysfunction or congestive heart failure;
- History of a previous non-disabling stroke, transient ischemic attack, or carotid endarterectomy;
- Urgent CABG intervention defined as the need to stay in the hospital (although the patient may be operated on within a normal scheduling routine);
- History of myocardial infarction that occurred more than 48 hours but less than 6 weeks prior to CABG surgery;
- Prior peripheral artery surgery or angioplasty;
- Moderate renal dysfunction defined as creatinine > 133 micromol/L (1.5 mg/dl), but < 250 micromol/L (2.8 mg/dl); and
- Presence of at least one asymptomatic carotid artery stenosis (>50%) either in one or two carotid arteries.

The first dose of study medication is administered at 3-10 hours prior to CABG surgery. In the event of surgery delay or rescheduling, a second pre-operative dose of P5P is administered so that all patients receive study medication 3-10 hours before surgery. Treatment continues for 30 days after surgery (post operative day (POD) 30). Patients receive follow-up evaluations up to and including postoperative day (POD) 4, on POD 30 and on POD 90.

Patients are measured for combined incidence of cardiovascular death, nonfatal myocardial infarction (MI), and nonfatal cerebral infarction up to and including post-operative day 30 (POD 30). Patients are also measured for nonfatal myocardial infarction alone.

All deaths without an identifiable non-cardiovascular cause are considered of cardiovascular origin.

Cerebral infarction (stroke) is defined clinically as any new sudden onset focal neurological deficit lasting at least 24 hours as assessed by a neurologist and after neuroimaging (computed tomography [CT] brain scan or magnetic resonance imaging [MRI]) excludes an intracerebral hemorrhage. All patients suspected of having a stroke or transient ischemic attack (TIA) receive a neurological examination (conducted by a neurologist or internist with expertise in cerebral vascular disease) within 24 hours of onset of symptoms. All patients suspected of having a stroke are submitted to cerebral imaging.

When determining whether a patient has myocardial infarction, the following definitions are used:
- A peak creatine kinase - myocardial band (CK-MB) above a certain threshold.
   Since different experts and different prior art clinical trials used different cut-offs for this threshold, three different thresholds were used to determine whether a patient had myocardial infarction -- a cut off threshold of 100 ng/ml (a peak CK-MB of 100 ng/ml or greater on days up to and including POD 4), a cut off threshold of 70 ng/ml, and a cut off threshold of 50 ng/ml was used;
- A new q-wave evidence of myocardial infarction along with CK-MB of 35 ng/ml or above on days up to and including POD 4;
- A peak CK-MB of 5x ULN (25 ng/ml) or above occurring after POD 4;
- A new q-wave evidence of myocardial infarction that was not present at POD 4; or
- A q-wave or non-q-wave myocardial infarction as identified by the investigator and confirmed by the Clinical Endpoint Committee.

### A. Myocardial Infarction Rates

### CK-MB cut-off of 100 ng/ml

Intravenous treatment of both 4.69 mg/day and 14.07 mg/day is highly effective in reducing instances of myocardial infarction.

### Using CK-MB cut-off of 70 ng/ml

Intravenous treatment of 4.69 mg/day is highly effective in reducing instances of myocardial infarction. Intravenous treatment of 14.07 mg/day is effective in reducing instances of myocardial infarction, but is not as effective as treatment of 4.69 mg/day.

### Using CK-MB cut-off of 50 ng/ml

Intravenous treatment of 4.69 mg/day is highly effective in reducing instances of myocardial infarction. Intravenous treatment of 14.07 mg/day is not effective in reducing instances of myocardial infarction.

### B. Combined Incident / Composite Endpoint Rates

Frequencies of combined incidences (including myocardial infarction, cerebral infarction, or death), by treatment group, are as follows:

### CK-MB cut-off of 100 ng/ml

Intravenous treatment of either 4.69 mg/day or 14.07 mg/day is highly effective in reducing total incidents. Intravenous treatment of 4.69 mg/day is marginally better at reducing total incidents as compared to treatment of 14.07 mg/day.

### CK-MB cut-off of 70 ng/ml

Intravenous treatment of either 4.69 mg/day or 14.07 mg/day is highly effective in reducing total incidents. Intravenous treatment of 4.69 mg/day is better at reducing total incidents than treatment of 14.07 mg/day.

### CK-MB cut-off of 50 ng/ml

Intravenous treatment of 4.69 mg/day is highly effective in reducing total incidents. Intravenous treatment of 14.07 mg/day does not reduce total incidents.

## Claims

1. A composition comprising about 250 mg of pyridoxal-5'-phosphate, and a pharmaceutically acceptable excipient.

2. A composition comprising about 750 mg ofpyridoxal-5'-phosphate, and a pharmaceutically acceptable excipient.

3. A composition comprising about 4.69 mg of pyridoxal-5'-phosphate, and a pharmaceutically acceptable excipient.

4. A composition comprising about 14.07 mg of pyridoxal-5'-phosphat, and a pharmaceutically acceptable excipient.

5. Use of 100 to 1000 mg of a compound selected from the group consisting of pyridoxine, pyridoxal-5'-phosphate, pyridoxal and pyridoxamine in the preparation of a medicament for the treatment or prevention of hypertrophy, organ ischemia, tissue ischemia, acute coronary syndrome, unstable angina, myocardial ischemia, conjestive heart failure, contractile dysfunction, ischemia reperfusion injury, or arrhythmia.

6. Use of 100 to 1000 mg of a compound selected from the group consisting of pyridoxine, pyridoxal-5'-phosphate, pyridoxal and pyridoxamine in the preparation of a medicament for the treatment or prevention of post-operative complications.

7. The use of claim 6, wherein the post-operative complication arises from an operation selected from the group consisting of bypass surgery, thrombolysis, CABG, and angioplasty.

8. The use of any one of claims 6 or 7, wherein the post-operative complication is hypertrophy, myocardial ischemia, organ ischemia, tissue ischemia, acute coronary syndrome, unstable angina, ischemia reperfusion injury, arrhythmia, conjestive heart failure, contractile dysfunction, cerebral infarction (stroke), or death.

9. The use of any one of claims 5-8, wherein between about 200 mg and about 300 mg of the compound is used.

10. The use of any one of claims 5-9, wherein about 250 mg of the compound is used.

11. The use of any one of claims 5-8, wherein about 750 mg of the compound is used.

12. Use of 1.88 mg to 18.76 mg of a compound selected from the group consisting of pyridoxine, pyridoxal-5'-phosphate, pyridoxal and pyridoxamine in the preparation of a medicament for the treatment or prevention of hypertrophy, myocardial ischemia, organ ischemia, tissue ischemia, acute coronary syndrome, unstable angina, conjestive heart failure, contractile dysfunction, ischemia reperfusion injury, or arrhythmia.

13. Use of 1.88 mg to 18.76 mg of a compound selected from the group consisting of pyridoxine, pyridoxal-5'-phosphate, pyridoxal and pyridoxamine in the preparation of a medicament for the treatment or prevention of post-operative complications.

14. The use of claim 13, wherein the post-operative complication arises from an operation selected from the group consisting of bypass surgery, thrombolysis, CABG, and angioplasty.

15. The use of any one of claims 13 or 14, wherein the post-operative complication is hypertrophy, myocardial ischemia, organ ischemia, tissue ischemia, acute coronary syndrome, unstable angina, ischemia reperfusion injury, arrhythmia, conjestive heart failure, contractile dysfunction, cerebral infarction (stroke), or death.

16. The use of any one of claims 12-15, wherein between about 3.76 mg and about 5.64 mg of the compound is used.

17. The use of any one of claims 12-16, wherein about 4.69 mg of the compound is used.

18. The use of any one of claims 12-15, wherein about 14.07 mg of the compound is used.

19. The use of any one of claims 5-18, wherein the compound is pyridoxal.

20. The use of any one of claims 5-18, wherein the compound is pyridoxine.

21. The use of any one of claims 5-18, wherein the compound is pyridoxamine.

22. The use of any one of claims 5-18, wherein the compound is pyridoxal-5'-phosphate.

23. A kit comprising:
(a) a pharmaceutical preparation for oral administration comprising a compound selected from the group consisting of pyridoxine, pyridoxal, pyridoxal-5'-phosphate, and pyridoxamine;
(b) instructions for the administration of said preparation,
said instructions specifying that said preparation should be administered in a dosage range of about 100 mg to about 1000 mg per day.

24. The kit of claim 23, wherein the instructions further specify that the preparation should be administered prior to or during a heart procedure selected from the group consisting of bypass surgery, thrombolysis, CABG, and angioplasty.

25. The kit of claim 23, wherein the instructions further specify that the preparation should be administered after a heart procedure selected from the group consisting of bypass surgery, thrombolysis, CABG, and angioplasty.

26. The kit of any one of claims 23-25 wherein the instructions further specify that said preparation should be administered in a dosage range of about 200 mg to about 300 mg per day.

27. The kit of any one of claims 23-26 wherein the instructions further specify that said preparation should be administered in a dosage of about 250 mg per day.

28. The kit of any one of claims 23-25 wherein the instructions further specify that said preparation should be administered in a dosage of about 750 mg per day.

29. A kit comprising:
(a) a pharmaceutical preparation for parenteral administration comprising a compound selected from the group consisting of pyridoxine, pyridoxal, pyridoxal-5'-phosphate, and pyridoxamine;
(b) instructions for the administration of said preparation,
said instructions specifying that said preparation should be administered in a dosage range of about 1.88 mg to about 18.76 mg per day.

30. The kit of claim 58, wherein the instructions further specify that the preparation should be administered prior to or during a heart procedure selected from the group consisting of bypass surgery, thrombolysis, CABG, and angioplasty.

31. The kit of claim 29, wherein the instructions further specify that the preparation should be administered after a heart procedure selected from the group consisting of bypass surgery, thrombolysis, CABG, and angioplasty.

32. The kit of any one of claims 58-60 wherein the instructions further specify that said preparation should be administered in a dosage range of about 3.76 mg to about 5.64 mg per day.

33. The kit of any one of claims 29-31 wherein the instructions further specify that said preparation should be administered in a dosage of about 4.69 mg per day.

34. The kit of any one of claims 29-31 wherein the instructions further specify that said preparation should be administered in a dosage of about 14.07 mg per day.
